# EUROPEAN PATENT APPLICATION

(11) **EP 1 707 113 A1**
(43) Date of publication of application: **04.10.2006**
(21) Application number: 06251735.4
(22) Date of filing: 29.03.2006
(51) Int. Cl.: A61B 5/00, G01N 21/64

(54) **Method for monitoring an implanted fluorescent light-emitting bead**

(30) Priority: 30.03.2005 US 93981
(71) Applicant: LifeScan, Inc., Milpitas, CA 95035 (US)
(72) Inventor: Kermani, Mahyar Z., Pleasanton, CA 94588 (US); Dale, Kimberly Delane, San Jose, CA 95128 (US)
(74) Representative: Mercer, Christopher Paul

(57) **Abstract**

A method for monitoring an implanted fluorescent light-emitting bead includes adhering and adhesive fluorescence measurement patch to a user's body. The method also includes monitoring the implanted fluorescent light-emitting bead implanted in the user's by emitting light from a light emitter included in the adhesive fluorescence measurement patch and detecting fluorescent light emitted from the fluorescent light-emitting bead with a light detector included in the adhesive fluorescence measurement patch.

## Description

### BACKGROUND OF THE INVENTION

### Field of the Invention

This application relates, in general, to medical devices and, in particular, to medical devices, kits and methods that employ fluorescence analytical techniques.

### Description of the Related Art

A variety of devices and methods for monitoring (e.g., detecting and/or measuring) analytes, such as glucose, in bodily fluids are employed by both medical personnel and laypersons. For example, the use of photometric-based and electrochemical-based devices and methods for monitoring blood glucose has become widely adopted for the treatment of diabetes.

Fluorescence analytical techniques designed for detecting and measuring analytes in bodily fluids have also been reported. For example, U.S. Patent No.s 5,342,789, 6,040,194 and 6,232,130 describe a variety of such techniques and related *in-vivo* sensors, including those adapted for the quantifying glucose concentration in blood or other bodily fluids.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:
FIG. 1 is a simplified schematic illustration depicting interaction between a fluorescent light-emitting bead, light emitter and light detector that is relevant to various embodiments of the present invention;
FIG. 2 is a simplified schematic illustration depicting interaction between a fluorescent light-emitting bead implanted in a user's body, a light emitter, and alight detector for detecting fluorescent light that is relevant to various embodiments of the present invention;
FIG. 3A is a simplified cross-sectional view of an adhesive fluorescence measurement patch according to an exemplary embodiment of the present invention removably adhered to a user's body;
FIG. 3B is a simplified schematic depicting the operative interaction of various electrical and optical components, including a light emitter and a light detector, suitable for use in the adhesive fluorescence measurement patch of FIG. 3A;
FIG. 4 is simplified perspective and partial cut-away view of the adhesive fluorescence measurement patch of FIG. 3A removably adhered to a user's body (i.e., a user's forearm);
FIG. 5 is a simplified top view depiction of an adhesive fluorescence measurement patch, according to another exemplary embodiment of the present invention;
FIG. 6 is a simplified bottom view depiction of the adhesive fluorescence measurement patch illustrated in FIG. 5;
FIG. 7 is a simplified bottom view depiction of an adhesive fluorescence measurement patch, according to yet another embodiment of the present invention;
FIG. 8 is a simplified bottom view depiction of an adhesive fluorescence measurement patch according to a further embodiment of the present invention;
FIG. 9 is a simplified top view depiction of the adhesive measurement patch of in FIG. 8 removably adhered to a user's body in a first position;
FIG. 10 is a simplified top view depiction of the adhesive measurement patch of in FIG. 8 removably adhered to a user's body in a second position;
FIG. 11 is a simplified top view depiction of an adhesive fluorescence measurement patch according to a still further exemplary embodiment of the present invention depicted in a first patch position and a second patch position;
FIG. 12 is a simplified top view depiction of an adhesive fluorescence measurement patch according to a yet further exemplary embodiment of the present invention depicted in a first patch position and a second patch position;
FIG. 13 is a simplified top view depiction of an adhesive fluorescence measurement patch according to an additional exemplary embodiment of the present invention depicted in a first patch position and a second patch position;
FIG. 14 is a simplified top view depiction of an adhesive fluorescence measurement patch according to a still another exemplary embodiment of the present invention depicted in a first patch position and a second patch position;
FIG. 15 is a flow diagram depicting stages in a process for monitoring a fluorescence bead implanted in a user's body according to an exemplary embodiment of the present invention; and
FIG. 16 is simplified perspective view of a fluorescence measurement band according to an exemplary embodiment of the present invention attached to a user's forearm.

### DETAILED DESCRIPTION OF PREFERRED EMBODIMENTS

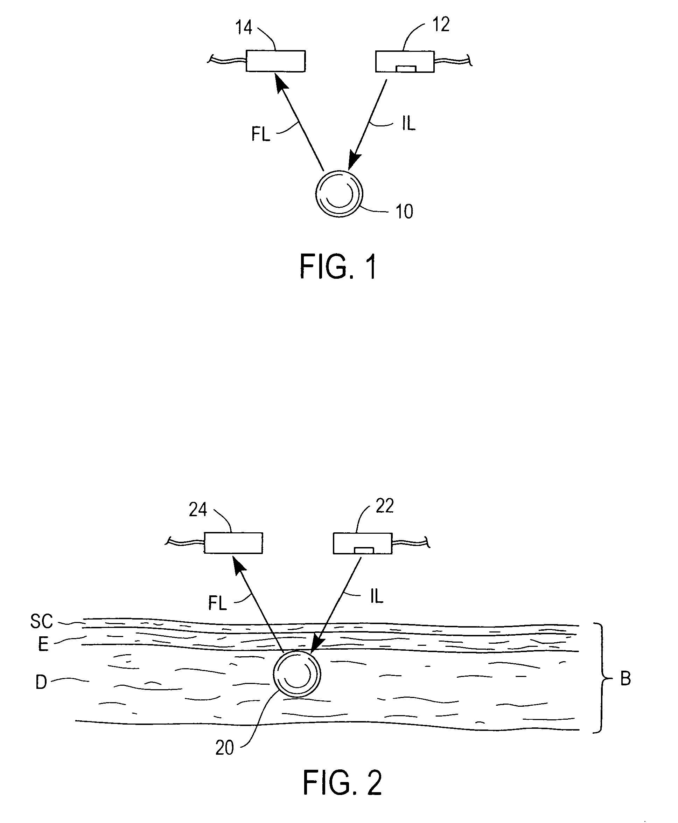

FIG. 1 is a simplified schematic illustration depicting interaction between a fluorescent light-emitting bead 10, light emitter 12 and light detector 14 that is relevant to various embodiments of the present invention. Fluorescent light-emitting bead 10 includes at least one fluorescent reactant (e.g., a fluorescent dye) that emits fluorescent light FL as a result of absorbing incident light IL (that has been emitted by light emitter 12), with the characteristics of the emitted fluorescent light FL being dependent on the concentration of an analyte that is in communication with (for example, in contact with) the fluorescent light-emitting bead. Fluorescent reactants that can be included in such a fluorescent light-emitting bead, and their behavior when in communication with an analyte, are described in U.S. Patent No.s 5,342,789, 6,040,194, and 6,232,130. Fluorescent light-emitting bead FB can also include an encapsulating material such as, for example, alginate.

FIG. 2 is a simplified schematic illustration depicting interaction between a fluorescent light-emitting bead 20 implanted in a user's body B, a light emitter 22, and a light detector 24 that is relevant to various embodiments of the present invention. The portion of user's body B depicted in FIG. 2 includes a Stratum Corneum portion SC, an Epidermis portion E and Dermis portion D.

As with fluorescent light-emitting bead 10, fluorescent light-emitting bead 20 includes at least one fluorescent reactant (e.g., a fluorescent dye) that emits fluorescent light FL as a result of absorbing incident light IL (that has been emitted by light emitter 22), with the characteristics of the emitted fluorescent light being dependent on the concentration of an analyte that is in communication with the fluorescent light-emitting bead.

FIG. 2 depicts fluorescent light-emitting bead 20 implanted in a user's body (B). In this circumstance, incident light IL and fluorescent light FL are of a wavelength(s) and intensity such that incident light IL is able to pass through the user's body to reach fluorescent light-emitting bead 10 and fluorescent light FL is able to pass through the user's body to reach light detector 24. Fluorescent light- emitting bead 20 includes at least one fluorescent reactant and is configured in such a way that a predetermined characteristic(s) of fluorescent light FL varies as a function of bodily fluid analyte concentration (e.g., glucose concentration) in the user's body B.

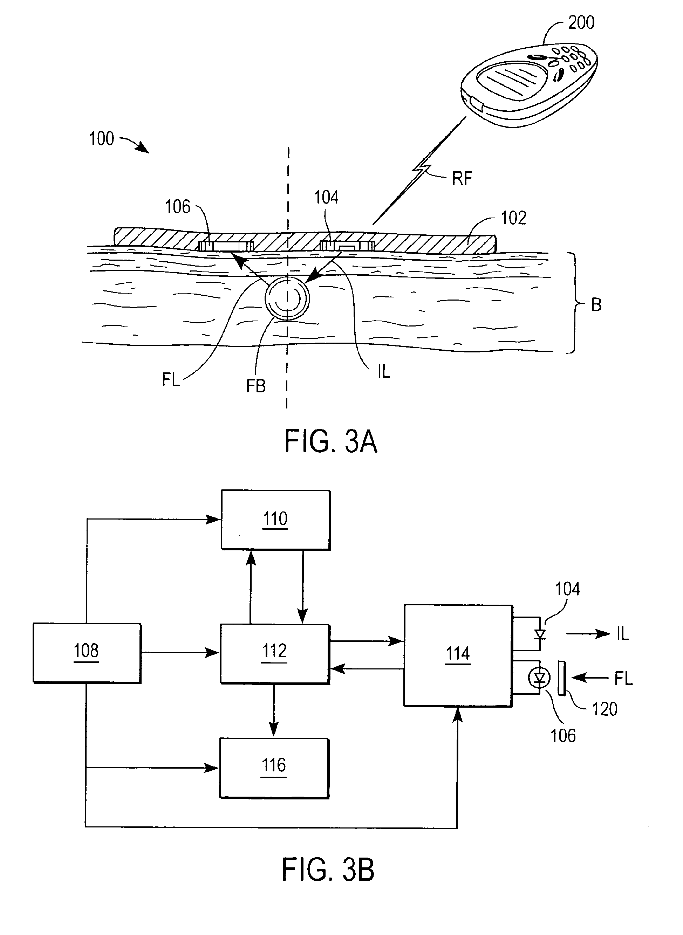

FIG. 3A is a simplified cross-sectional view of an adhesive fluorescence measurement patch 100 for use with a fluorescent light-emitting bead FB implanted within a user's body B, that includes a Stratum Corneum portion SC, an Epidermis portion E and Dermis portion D, according to an embodiment of the present invention. In FIG. 3A, adhesive fluorescence measurement patch 100 is removably adhered to a user's body B and in communication with a remote module 200 via radio-frequency signals RF. Adhesive fluorescence measurement patch 100 includes an adhesive sheet 102 configured for removable adhesion to user's body B, a light emitter 104 attached to adhesive sheet 102, and alight detector 106 also attached to adhesive sheet 102.

Fluorescent light-emitting bead FB can be implanted, for example, in the range of approximately 1mm to 4mm below the surface of a user's skin. In addition, light emitter 104 and light detector 106 can be located, for example, in the range of 0mm to 10mm above the surface of the user's skin when adhesive fluorescence measurement patch 100 is adhered to the user's body B (i.e., adhered to the user's skin).

For the sake of simplicity, FIG. 3A depicts adhesive fluorescence measurement patch 100 as including only an adhesive sheet, light emitter and light detector. However, once apprised of the present disclosure, one skilled in the art will recognize that adhesive fluorescence measurement patches according to the present invention can include various other components, electrical and/or optical, that provide for suitable and beneficial operation. In this regard, FIG. 3B is a simplified schematic diagram depicting the operative interaction of various electrical and optical components, including a light emitter 104 and a light detector 106, suitable for use in the adhesive fluorescence measurement patch of FIG. 3A. In FIG. 3B, elements or other items common with FIG. 3A are identically labelled.

As depicted in FIG. 3B, the electrical and optical components include a power module 108, an RF transceiver module 110, a micro-controller module 112, a driver/amplifier module 114, a buzzer module 116 (for providing feedback to a user) and an optical filter module 120. Light emitter 104 can be, for example, an LED 525nm wavelength light emitter such as SMD LED part number LTST-C903TGKT available from Lite-On Corp. Light detector 106 can be, for example, light detector part number S 8745-01 available from Hamamatsu. Optical filter module 120 can include, for example, 600nm and 700nm band pass filters. Micro-controller module 112 can be, for example, an MSP 430 series micro-controller available from Texas Instruments. Power module 108 can be, for example, a rechargeable or non-rechargeable battery module. If desired, all the electrical and optical components depicted in FIG. 3B can be mounted on a printed circuit board (PCB) and the PCB attached to adhesive sheet 102.

In addition, once apprised of the present disclosure, one skilled in the art will recognize that adhesive fluorescence measurement patches according to embodiments of the present invention could be readily modified for use with suitable fluorescent light-emitting devices other than a fluorescent light-emitting bead. For example, such adhesive fluorescence measurement patches could be used with fluorescent injected oils or fluorescent tattoos as described in U.S. Patent No. 5,342,789.

In FIG. 3A, fluorescent light-emitting bead FB is implanted in user's body B, and contains at least one fluorescent reactant that emits fluorescent light FL as a result of absorbing incident light IL. In addition, a characteristic(s) of fluorescent light FL varies as a function of analyte concentration in contact with fluorescent light-emitting bead FB. Therefore, adhesive fluorescence measurement patch 100, in conjunction with fluorescent light-emitting bead FB and remote module 200, can be used for measuring the concentration of an analyte (e.g., blood glucose) in the bodily fluid of a user's body.

Referring again to FIG. 3A, an imaginary optical axis X of adhesive fluorescence measurement patch 100 is depicted by a broken line. Light emitter 104 and light detector 106 are attached to adhesive sheet 102 in a predetermined relationship relative to imaginary optical axis X. In addition, imaginary optical axis X is positioned in a predetermined juxtaposition to the fluorescent light-emitting bead FB when the adhesive fluorescence measurement patch is removably adhered to a user's body (as in FIG. 3A). The predetermined juxtaposition of imaginary optical axis X and fluorescent light-emitting bead FB will typically be associated with a suitable alignment tolerance in the range of, for example, +/- 1mm to +/- 2mm.

The predetermined relationship of light emitter 104 and light detector 106 with imaginary optical axis X and the predetermined juxtaposition of imaginary optical axis X with the fluorescent light-emitting bead FB provide for (i) emitted incident light IL from light emitter 104 to be incident on, and absorbed by, fluorescent light-emitting bead FB and (ii) fluorescent light FL emitted by fluorescent light-emitting bead FB to be detected by light detector 106 (the emitted light IL and fluorescent light FL are, for the sake of simplicity, depicted as arrows in FIG. 3 (as well as in FIGs. 1 and 2)). Therefore, adhesive fluorescence measurement patch 100 can be readily adhered to user's body B in a position that provides for incident light IL to operatively reach fluorescent light-emitting bead FB and for fluorescent light FL to operatively reach light detector 106. Since light emitter 104 and light detector 106 are securely attached to adhesive fluorescence measurement patch 102 in a proper predetermined relationship to imaginary optical axis X, an operable alignment of light emitter 104 and light detector 106 with an implanted fluorescent light-emitting bead FB is easily obtained and maintained during use.

It should be noted that although FIG. 3A depicts light emitter 104 and light detector 106 as being symmetrically disposed about imaginary optical axis X, such symmetry is not necessarily required. In addition, the predetermined relationship of light emitter 104 and light detector 106 with imaginary optical axis X, as well as the predetermined juxtaposition of imaginary optical axis X with the fluorescent light-emitting bead FB, can be such the amount of reflected light from the fluorescent light-emitting bead received by the light detector is relatively minimized while the amount of fluorescent light received by the light detector is relatively maximized.

Adhesive sheet 102 can be any suitable adhesive sheet known to those of skill in the art including, for example, adhesive sheets that include commercially available pressure sensitive adhesives. Furthermore, adhesive sheets employed in embodiments of the present invention can include a top layer and at least one adhesive lower layer disposed on at least a portion of the top layer.

The top layer and adhesive lower layer(s) employed in the adhesive sheet can be any suitable combination of single-sided adhesive layers, double-sided adhesive layers, transfer adhesive layers and non-adhesive layers. The single-sided and double-sided adhesive layers can be pressure sensitive, in that they removably adhere to a surface of a user's body when pressure is applied. Typical pressure sensitive adhesive layers include those based on acrylics, natural rubber, synthetic rubber and silicone polymers. Suitable pressure sensitive adhesive layers are commercially available from, for example, Adhesives Research, Inc., of Glen Rock, Pennsylvania under the commercial name ARcare® .

The top layer and adhesive lower layer(s) of an adhesive sheet can be clear or opaque, and are typically flexible. The top layer and adhesive lower layer(s) can be made, for example, from an extruded or cast polymer film, or can be made using woven or non-woven fabric and can be elastic, or inelastic. In addition, they can be made from any suitable material, including, for example polyester, polycarbonate, polystyrene, polypropylene, polyethylene, acrylonitrile butadiene styrene, polyurethane, silicone, and woven or non-woven fabrics. Suitable polymer films and fabrics can be purchased, for example, from Tekra Corporation of New Berlin, Wisconsin.

If desired, one or more release liners can be employed to cover all or a portion of adhesive sheets employed in embodiments of the present invention. Such release liners are typically made by, for example, siliconizing polyester, polyethylene, polypropylene or paper. Release liners can also be manufactured by treating the surface of a suitable material with a fluorocarbon-based compound. Prior to use of an adhesive fluorescence measurement patch, one or all of the release liners are pealed off of the adhesive sheet. Suitable release liners are commercially available from, for example, Rexam Release, of Bedford Park, Illinois.

The adhesive sheet employed in embodiments of the present invention can be any suitable thickness. However, a typical non-limiting thickness range is from 0.0005 inches to 0.040 inches (excluding the thickness of the light emitter and light detector that are attached to the adhesive sheet). A major surface of the adhesive fluorescence measurement patch (i.e., the surface facing a user's body when the adhesive fluorescence measurement patch is adhered) has a surface area, for example, in the range of from 0.40 square inches to 4 square inches.

Any suitable light emitter 104 and suitable light detector 106 known to one skilled in the art can be employed in adhesive fluorescence measurement patches according to embodiments of the present invention. Suitable light emitters can be, for example, light emitting diodes (e.g., light emitting diodes commercially available from Lite-On Technology Corporation of Milpitas, California). Suitable light detectors can be, for example, photodiodes (e.g., photodiodes commercially available from Hamamatsu Corporation of Bridgewater, New Jersey).

In FIG. 3A, adhesive fluorescence measurement patch 100 is depicted as in communication with remote module 200 via radio frequency signals RF. However, once apprised of the present disclosure, one skilled in the art will recognize that other suitable means of providing communication between an adhesive fluorescence measurement patch and a remote module can be employed, including wired communication.

Remote module 200 can have any suitable capabilities, including the capability to control of light emitter 104 and light detector 106 and the capability to process communications received from adhesive fluorescence measurement patch 100. For example, remote module 200 can have the capability to continuously or intermittently correlate fluorescent light detected by light detector 106 to analyte concentration and to then employ the correlation to control other devices, such as an insulin pump. Suitable remote controllers, as can be modified by one skilled in the art for use in embodiments of the present invention, are described in international publication WO 03/071930 A2.

One skilled in the art will recognize that adhesive fluorescence measurement patch 100 is symmetrically shaped (i.e., circular in shape) in one dimension about imaginary optical axis X. However, as described below, adhesive fluorescence measurement patches according to other embodiments of the present invention can be non-symmetrically shaped (e.g., square, rectangular, oval or triangular shaped) about their imaginary optical axis.

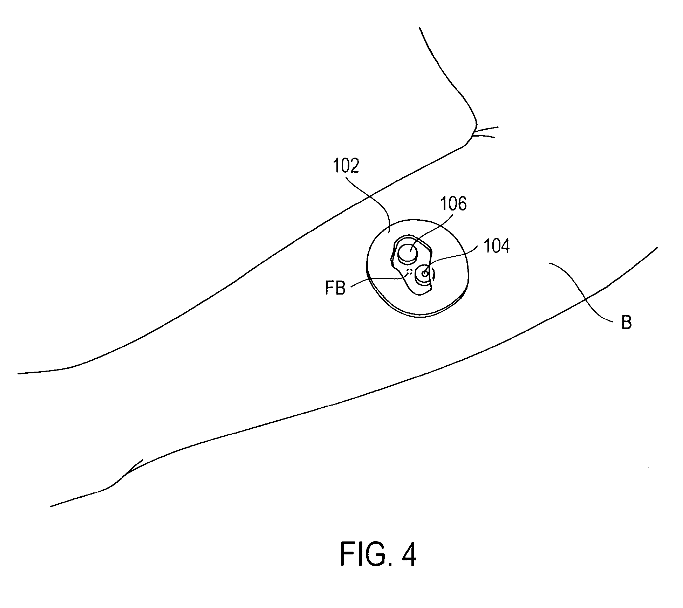

FIG. 4 is simplified perspective and partial cut-away view of the adhesive fluorescence measurement patch of FIG. 3 removably adhered to a user's body B (i.e., a user's forearm). In the embodiment of FIGs. 3 and 4, imaginary optical axis X is aligned with fluorescent light-emitting bead FB. In addition, since imaginary optical axis X passes through the center of adhesive fluorescence measurement patch 100, adhesive fluorescence measurement patch 100 is itself centered above fluorescent light-emitting bead FB when removably adhered to user's body B. However, once apprised of the present disclosure, one skilled in the art will recognize that adhesive fluorescence measurement patches according to the present invention need not necessarily be centered above a fluorescent light-emitting bead FB, as long as the positioning of the adhesive fluorescence measurement patch provides for (i) emitted incident light IL from light emitter 104 to be incident on, and absorbed by, fluorescent light-emitting bead FB and (ii) fluorescent light FL emitted by fluorescent light-emitting bead FB to be detected by light detector 106.

Since adhesive fluorescence measurement patch 100 is adhered (albeit removably) to user's body B, light emitter 104 and light detector 106 remain essentially stationary relative to fluorescent light-emitting bead FB.

When adhered to a user's body, adhesive fluorescence measurement patch 100 can be used, for example, to continuously monitor blood glucose concentration within the user's body. In this circumstance, adhesive fluorescence measurement patch 100 can be removed and replaced, as needed, during the lifetime of fluorescent light-emitting bead FB (which can range from days to years).

An analytical fluorescence measurement kit according to exemplary embodiments of the present invention includes an adhesive fluorescence measurement patch (as described herein), a fluorescent light-emitting bead (also as described herein) and a remote module (as described above). For example, an analytical fluorescence measurement kit can include adhesive fluorescence measurement patch 100, fluorescent light-emitting bead FB and remote module 200 of FIG. 2. However, analytical fluorescence measurement kits according to exemplary embodiments of the present invention can include a fluorescence measurement band (as described below) rather than an adhesive fluorescence measurement patch.

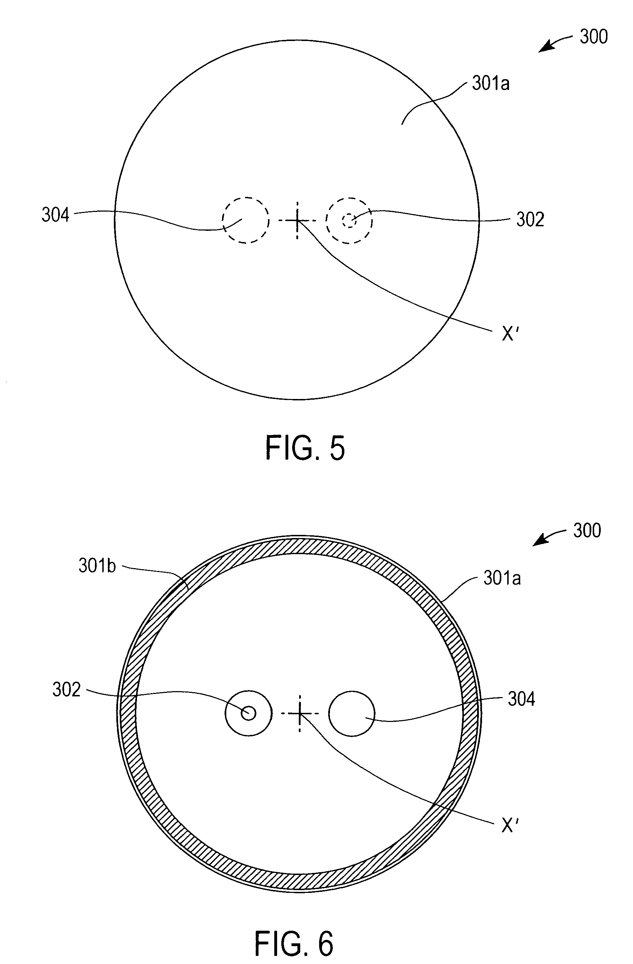

FIGs. 5 and 6 are a simplified top and bottom view depictions, respectfully, of an adhesive fluorescence measurement patch 300 for use with a fluorescent light-emitting bead implanted within a user's body according to another exemplary embodiment of the present invention. Adhesive fluorescence measurement patch 300 includes an adhesive sheet composed of upper layer 301a and adhesive lower layer 301b. Adhesive lower layer 301b is configured for removably adhering adhesive fluorescence measurement patch 300 to the user's body.

Adhesive fluorescence measurement patch 300 also includes a light emitter 302 attached to upper layer 301a and a light detector 304 also attached to upper layer 301a. Light emitter 302 and light detector 304 are depicted by dashed lines in FIG. 5 to represent that they are not visible in such a top view. Adhesive fluorescence measurement patch 300 is symmetric about imaginary optical axis X'.

Adhesive lower layer 301 b is disposed around the perimeter of upper layer 301 a. It is postulated without being bound that the disposition of adhesive lower layer 301b around only the perimeter of upper layer 301a provides for secure adhesion while reducing irritation of a user's body in comparison to an adhesive lower layer disposed on the entirety of upper layer 301 a. Adhesive lower layer 301b can be deposited onto upper layer 301a by any suitable technique including, for example, screen printing, ink jetting, slot coating and lamination.

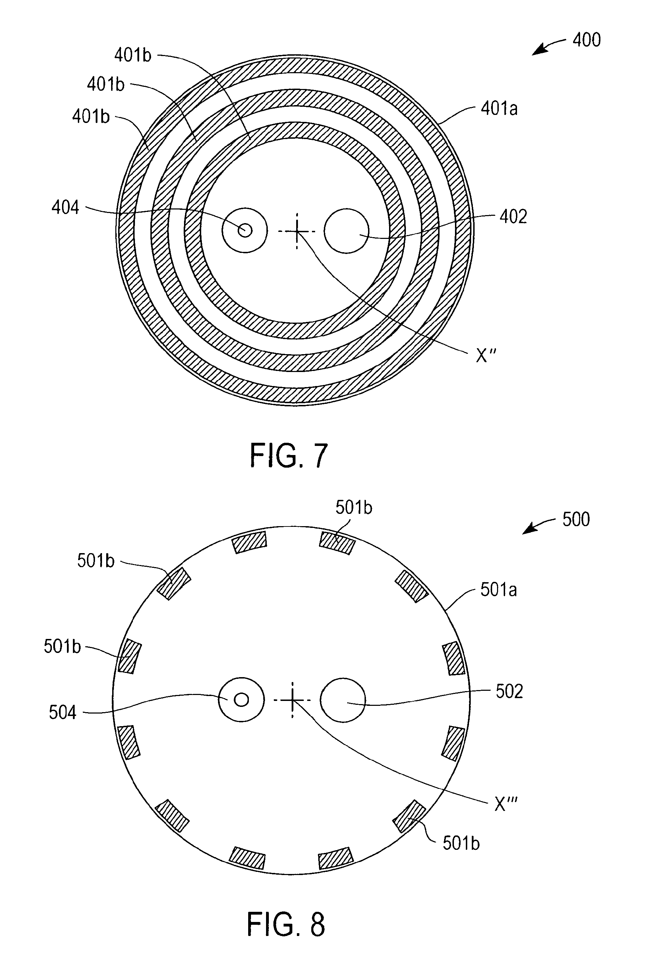

FIG. 7 is a simplified bottom view depiction of an adhesive fluorescence measurement patch 400 for use with a fluorescent light-emitting bead implanted within a user's body according to another exemplary embodiment of the present invention. Adhesive fluorescence measurement patch 400 includes an adhesive sheet with an upper layer 401 a and a segmented adhesive lower layer 401b. In the embodiment of FIG. 7, segmented adhesive lower layer 401b includes three concentric circular segments.

Adhesive fluorescence measurement patch 400 also includes a light emitter 402 attached to upper layer 401 a and a light detector 404 that is also attached to upper layer 401a. Adhesive fluorescence measurement patch 400 is symmetric about imaginary optical axis X".

If desired, each segment of the segmented adhesive lower layer 401b can be optionally provided with a release liner, allowing a user to select which segment of segmented adhesive lower layer 401b will be employed to removably adhere adhesive fluorescence measurement patch 400 to a user's body. By selecting different concentric circular segments of segmented adhesive lower layer 401b during different time periods of use, a user can beneficially reduce the risk of skin irritation and/or maceration. Such a selection can be accomplished by removing the release liner that covers the particular segment that a user desires to use. Although, for illustration and explanation purpose, FIG. 7 depicts a segmented adhesive lower layer with three segments, any suitable numbers of segments can be employed.

FIG. 8 is a simplified bottom view depiction of an adhesive fluorescence measurement patch 500 for use with a fluorescent light-emitting bead implanted within a user's body according to another exemplary embodiment of the present invention. Adhesive fluorescence measurement patch 500 includes an adhesive sheet with an upper layer 501a and a segmented adhesive lower layer 501b. Although, for illustration and explanation purposes, FIG. 8 depicts segmented adhesive lower layer 501b with twelve segments disposed around the perimeter of upper layer 501 a, any suitable numbers of segments can be employed.

Adhesive fluorescence measurement patch 500 also includes a light emitter 502 attached to upper layer 501 a and a light detector 504 that is also attached to upper layer 501a. Adhesive fluorescence measurement patch 500 is symmetric about imaginary optical axis X"'.

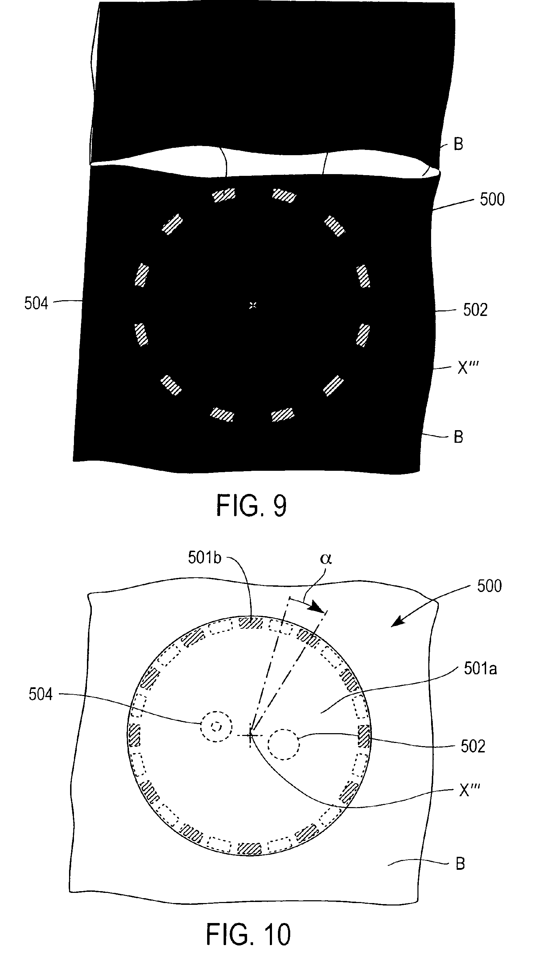

FIG. 9 is a simplified top view depiction of adhesive fluorescence measurement patch 500 of FIG. 8 adhered to a user's body B in a first position. FIG. 10 is a simplified top view depiction of the adhesive fluorescence measurement patch 500 adhered to user's body B in a second position wherein adhesive fluorescence measurement patch 500 has been rotated through angle α around imaginary optical axis X"'. In FIG. 10, dashed lines indicate the location where segmented adhesive lower layer 501b was adhered to user's body B in the first position of FIG. 9. The contact locations between segmented adhesive lower layer 501b (also referred to as adhesive contact locations) and user's body B in the first position are non-coincidental (i.e., do not overlap with) the contact locations between segmented adhesive lower layer 501b and a user's body B when the adhesive fluorescence measurement patch 500 is in the second position.

After a period of time (e.g., up to several days), the portion of a user's body B that is in contact with an adhesive layer (e.g., segmented adhesive lower layer 501b) can become irritated and/or damaged. It can be, therefore, desirable to remove the segmented adhesive lower layer from contact with those portions of the user's body to allow for recovery from the irritation or damage. In this regard, it should be noted that a fluorescent light-emitting bead is fixed in position once implanted in a user's body, and cannot be easily removed or repositioned.

However, by rotating adhesive fluorescence measurement patch 500 from the first position (of FIG. 9) to the second position (of FIG. 10), portions of a user's body that have become irritated or damaged from contact with segmented adhesive lower layer 501 b in the first position can be relieved from further irritation or damage and can recover. Rotation from the first position to the second position can also be accomplished by removing an adhesive fluorescence measurement patch 500 from the first position and replacing it with another adhesive fluorescence measurement patch (e.g., an unused adhesive fluorescence measurement patch) in the second position. In this regard, it should be noted that imaginary optical axis X"' is identically positioned relative to a fluorescent light-emitting bead implanted in user's body B in both the first position and the second position. Moreover, since light emitter 502 and light detector 504 are attached to upper layer 501a, they maintain a predetermined relationship relative to imaginary optical axis X'" in both the first position and the second position.

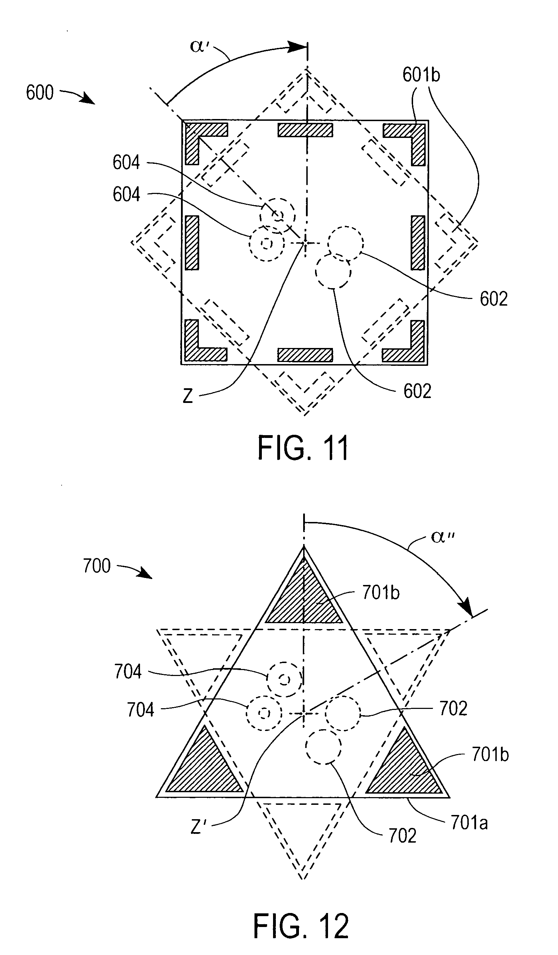

FIG. 11 is a simplified top view depiction of an adhesive fluorescence measurement patch 600 for use with a fluorescent light-emitting bead implanted within a user's body according to another exemplary embodiment of the present invention. Adhesive fluorescence measurement patch 600 (which is square in shape) includes an adhesive sheet with an upper layer 601 a and a segmented adhesive lower layer 601b. Although, for illustration and explanation purposes, FIG. 11 depicts adhesive fluorescence measurement patch to be square in shape and segmented adhesive lower layer 601b to have eight segments disposed around the perimeter of upper layer 601a, any suitable shape and number of segments can be employed.

FIG. 11 depicts adhesive fluorescence measurement patch in a first position using solid lines for elements 601a and 601b and in a second position using dashed lines for elements 601a and 601b. In the second position, adhesive fluorescence measurement patch 600 has been rotated about imaginary optical axis Z by angle α'.

Adhesive fluorescence measurement patch 600 also includes a light emitter 602 attached to upper layer 601a and a light detector 604 that is also attached to upper layer 601a. Light emitter 602 and light detector 604 are depicted with dashed lined for both the first and second positions since they are not visible in the view of FIG. 11.

In the embodiment of FIG. 11, contact locations between segmented adhesive lower layer 601b and user's body B in the first position are non-coincidental (i.e., do not overlap with) the contact locations between segmented adhesive lower layer 601 b and a user's body B when the adhesive fluorescence measurement patch 600 is in the second position.

FIG. 12 is a simplified top view depiction of an adhesive fluorescence measurement patch 700 for use with a fluorescent light-emitting bead implanted within a user's body according to another exemplary embodiment of the present invention. Adhesive fluorescence measurement patch 700 (which is triangular in shape) includes an adhesive sheet with an upper layer 701 a and a segmented adhesive lower layer 701 b. Although, for illustration and explanation purposes, FIG. 12 depicts adhesive fluorescence measurement patch to be triangular in shape and segmented adhesive lower layer to have three segments disposed around the perimeter of upper layer 701a, any suitable shape and number of segments can be employed.

FIG. 12 depicts adhesive fluorescence measurement patch in a first position using solid lines for elements 701 a and 701 b and in a second position using dashed lines for elements 701a and 701b. In the second position, adhesive fluorescence measurement patch 700 has been rotated about imaginary optical axis Z' by angle α".

Adhesive fluorescence measurement patch 700 also includes a light emitter 702 attached to upper layer 701a and a light detector 704 that is also attached to upper layer 701a. Light emitter 702 and light detector 704 are depicted with dashed lined for both the first and second positions since they are not visible in the view of FIG. 12.

In the embodiment of FIG. 12, contact locations between segmented adhesive lower layer 701b and user's body B in the first position are non-coincidental (i.e., do not overlap with) the contact locations between segmented adhesive lower layer 701b and a user's body B when the adhesive fluorescence measurement patch 700 is in the second position.

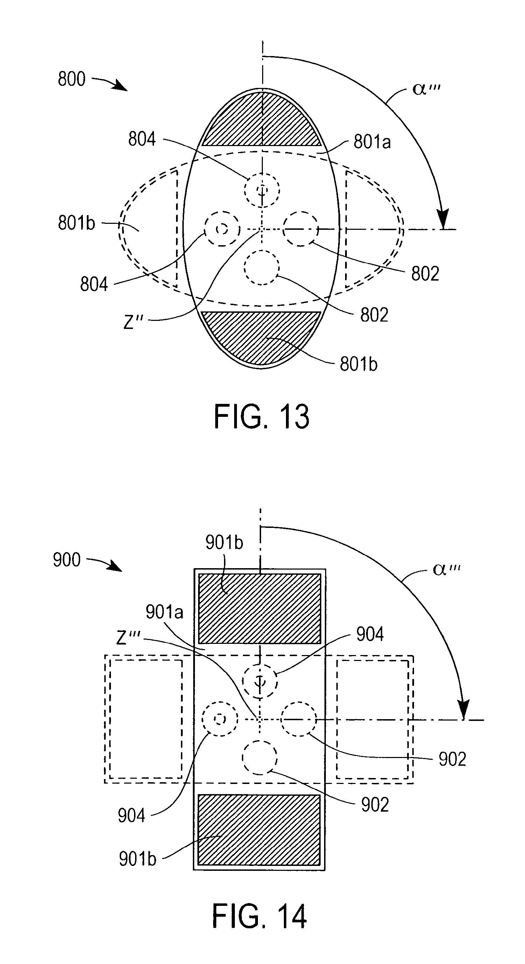

FIG. 13 is a simplified top view depiction of an adhesive fluorescence measurement patch 800 for use with a fluorescent light-emitting bead implanted within a user's body according to another exemplary embodiment of the present invention. Adhesive fluorescence measurement patch 800 (which is oval in shape) includes an adhesive sheet with an upper layer 801 a and a segmented adhesive lower layer 801 b. Although, for illustration and explanation purposes, FIG. 13 depicts adhesive fluorescence measurement patch to be oval in shape and segmented adhesive lower layer 801b to have two segments disposed at opposing ends of upper layer 801a, any suitable shape and number of segments can be employed.

FIG. 13 depicts adhesive fluorescence measurement patch in a first position using solid lines for elements 801a and 801b and in a second position using dashed lines for elements 801a and 801b. In the second position, adhesive fluorescence measurement patch 800 has been rotated about imaginary optical axis Z" by angle α"'.

Adhesive fluorescence measurement patch 800 also includes a light emitter 802 attached to upper layer 801 a and a light detector 804 that is also attached to upper layer 801a. Light emitter 802 and light detector 804 are depicted with dashed lined for both the first and second positions since they are not visible in the view of FIG. 13.

As is evident from FIG. 13, contact locations between segmented adhesive lower layer 801b and user's body B in the first position are non-coincidental (i.e., do not overlap with) the contact locations between segmented adhesive lower layer 801b and a user's body B when the adhesive fluorescence measurement patch 800 is in the second position.

FIG. 14 is a simplified top view depiction of an adhesive fluorescence measurement patch 900 for use with a fluorescent light-emitting bead implanted within a user's body according to another exemplary embodiment of the present invention. Adhesive fluorescence measurement patch 900 (which is rectangular in shape) includes an adhesive sheet with an upper layer 901a and a segmented adhesive lower layer 901b. Although, for illustration and explanation purposes, FIG. 14 depicts adhesive fluorescence measurement patch to be rectangular in shape and segmented adhesive lower layer to have two segments disposed at opposing ends of upper layer 901a, any suitable shape and number of segments can be employed.

FIG. 14 depicts adhesive fluorescence measurement patch in a first position using solid lines for elements 901a and 901b and in a second position using dashed lines for elements 901a and 901b. In the second position, adhesive fluorescence measurement patch 900 has been rotated about imaginary optical axis Z"' by angle α"".

Adhesive fluorescence measurement patch 900 also includes a light emitter 902 attached to upper layer 901 a and a light detector 904 that is also attached to upper layer 901 a. Light emitter 902 and light detector 904 are depicted with dashed lined for both the first and second positions since they are not visible in the view of FIG. 14.

In the embodiment of FIG. 14, contact locations between segmented adhesive lower layer 901b and user's body B in the first position are noncoincidental (i.e., do not overlap with) the contact locations between segmented adhesive lower layer 901b and a user's body B when the adhesive fluorescence measurement patch 900 is in the second position.

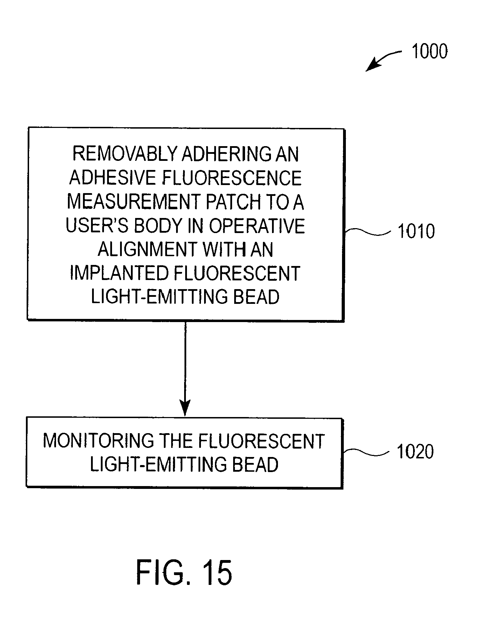

FIG. 15 is a flow diagram depicting stages in a method 1000 for monitoring a fluorescent light-emitting bead implanted in a user's body according to an exemplary embodiment of the present invention. Method 1000 includes removably adhering a adhesive fluorescence measurement patch to the user's body in a first position such that the adhesive fluorescence measurement patch is in operative alignment with the fluorescent light-emitting bead, as set forth in step 1010. The adhesive fluorescence measurement patch employed in step 1010 can be any suitable adhesive fluorescence measurement patch described herein.

Subsequently, at step 1020 of FIG. 15, the fluorescent light-emitting bead implanted in the user's body is monitored by emitting incident light from the light emitter and detecting fluorescent light emitted from the fluorescent light-emitting bead with the light detector. If desired, method 1000 can also include removing the adhesive fluorescence measurement patch from the first position and readhering an adhesive fluorescence measurement patch (either a new, i.e., unused, adhesive fluorescence measurement patch or the same adhesive fluorescence measurement patch that was removed) in a second position. In this circumstance, adhesive contact locations of the first position are non-coincidental (i.e., do not overlap with) with adhesive contact positions of the second position.

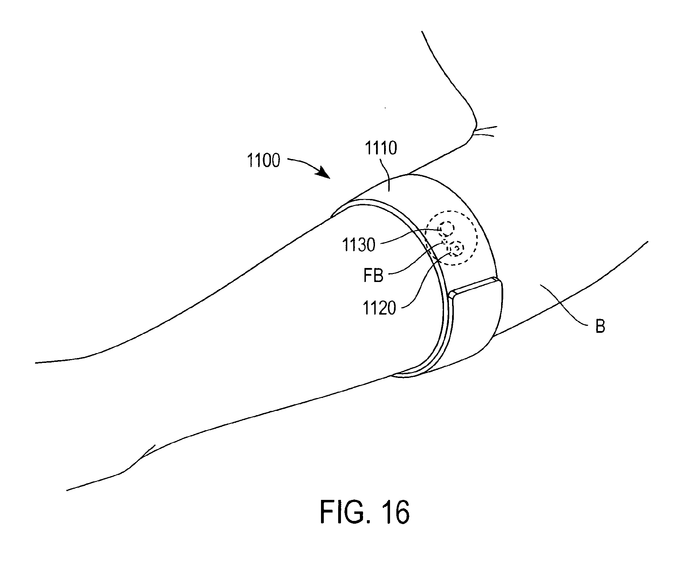

FIG. 16 is an illustration of a fluorescence measurement band 1100 for use with a fluorescent light-emitting bead implanted within a user's body according to an exemplary embodiment of the present invention. FIG. 16 depicts fluorescence measurement band 1100 securely and removably positioned about a portion of a user's body (namely, a user's forearm).

Fluorescence measurement band 1100 includes a band 1110 configured for secure and removable positioned about a portion of the user's body, a light emitter 1120 attached to the band, and a light detector 1130 attached to the band. Such positioning can be achieved, for example, by forming fluorescence measurement band 1100 at least partially of (i) self fastening materials, such as Velcro® brand hook and loop fasteners (sold by Velcro USA Inc. of Manchester, New Hampshire, and Coban™ Self-Adherent Wrap, sold by 3M Company of St. Paul, Minnesota) or (ii) of an elastic material. In addition, conventional fasteners can be employed to securely and removably position fluorescence measurement bands according to the present invention about a portion of a user's body.

Light emitter 1120 is configured for emitting light that is absorbed by fluorescent light-emitting bead FB. In addition, light detector 1130 is configured for detecting fluorescent light emitted by the fluorescent light-emitting bead FB. As illustrated in FIG. 16, fluorescence measurement band 1100 is positioned over fluorescent light-emitting bead FB, such that incident light from light emitter 1120 can reach fluorescent light-emitting bead FB, and fluorescent light from fluorescent light-emitting bead FB can reach light detector 1130.

It should be understood that various alternatives to the embodiments of the invention described herein may be employed in practising the invention. It is intended that the following claims define the scope of the invention and that structures and methods within the scope of these claims and their equivalents be covered thereby.

## Claims

1. A method for monitoring a fluorescent light-emitting bead implanted in a user's body, the method comprising:
removably adhering a adhesive fluorescence measurement patch to the user's body in a first position that is in operative alignment with the fluorescent light-emitting bead, wherein the adhesive fluorescence measurement patch includes:
at least one adhesive sheet configured for removable adhesion to the user's body;
a light emitter attached to the at least one adhesive sheet, the light emitter configured for emitting light that is absorbed by the fluorescent light emitting bead; and
a light detector attached to the at least one adhesive layer, the light detector configured for detecting fluorescent light emitted by the
fluorescent light-emitting bead; and
monitoring the fluorescent light-emitting bead implanted in the user's body by emitting light from the light emitter and detecting fluorescent light emitted from the fluorescent light-emitting bead with the light detector.

2. The method of claim 1 further comprising the steps of:
removing the adhesive fluorescence measurement patch from the first position and readhering an adhesive fluorescence measurement patch in a second position.

3. The method of claim 2, wherein the removing step includes re-adhering an unused adhesive fluorescence measurement patch in the second position.

4. The method of claim 2, wherein the removing step includes re-adhering, in the second position, the adhesive fluorescence measurement patch that had been removed from the first position.

5. The method of any one of claims 2 to 4, wherein adhesive contact locations of the first position are non-coincidental with adhesive contact positions of the second position.

6. The method of any one of claims 1 to 5, wherein the light emitter and light detector are attached to the at least one adhesive sheet in predetermined relationship relative to an imaginary optical axis of the adhesive fluorescence measurement patch, the imaginary optical axis being positioned in a predetermined juxtaposition to the fluorescent light-emitting bead when the adhesive fluorescence measurement patch is removably adhered to a user's body.

7. The method of any one of claims 1 to 6, wherein the monitoring step includes employing a remote module to control the light emitter and the light detector.

8. The method of any one of claims 1 to 7, wherein the monitoring step includes employing a remote module to process communications from the adhesive fluorescence measurement patch.

9. The method of any one of claims 1 to 8, wherein the at least one adhesive sheet includes a top layer and at least one segmented adhesive lower layer disposed on at least a portion of the top layer, with the adhesive lower layer configured for removably adhering the adhesive fluorescence measurement patch to the user's body.
